(19) **Europäisches Patentamt**
**European Patent Office**
**Office européen des brevets**

(11) Veröffentlichungsnummer : **0 475 044 B1**

(12) # EUROPÄISCHE PATENTSCHRIFT

(45) Veröffentlichungstag der Patentschrift :
**06.04.94 Patentblatt 94/14**

(51) Int. Cl.[5] : **C07C 303/22**

(21) Anmeldenummer : **91112547.4**

(22) Anmeldetag : **26.07.91**

(54) **Verfahren zur Herstellung von Aminoarylsulfonsäuren.**

(30) Priorität : **08.08.90 DE 4025131**

(43) Veröffentlichungstag der Anmeldung :
**18.03.92 Patentblatt 92/12**

(45) Bekanntmachung des Hinweises auf die
Patenterteilung :
**06.04.94 Patentblatt 94/14**

(84) Benannte Vertragsstaaten :
**CH DE FR GB IT LI**

(56) Entgegenhaltungen :
**EP-A- 0 000 634**
**EP-A- 0 146 889**
**DE-A- 2 703 076**
**GB-A- 1 499 589**
**GB-A- 1 576 608**

(73) Patentinhaber : **BAYER AG**
**D-51368 Leverkusen (DE)**

(72) Erfinder : **Steffan, Guido, Dr.**
**Herzogenfeld 52**
**W-5068 Odenthal (DE)**

EP 0 475 044 B1

## Beschreibung

Die Erfindung betrifft ein neues Verfahren zur Herstellung von Aminoarylsulfonsäuren durch katalytische Hydrierung von Nitroarylsulfonsäuren.

Es sind bereits verschiedene Verfahren zur Herstellung von Aminoarylsulfonsäuren durch katalytische Hydrierung von Nitroarylsulfonsäuren bekannt (siehe z.B. DE-PS 27 03 076, GB-PS 15 76 608 und EP-A 000 634). Von den in diesen Druckschriften beschriebenen Verfahren sind in der Praxis die Verfahren besonders bevorzugt, in denen als Hydrierkatalysatoren die preiswerten Nickel-Katalysatoren wie Raney-Nickel und Raney-Nickel/Eisen verwendbar sind und die Verwendung der teuren Edelmetallkatalysatoren Palladium und Platin nicht erforderlich ist.

Es hat sich jedoch gezeigt, daß die in der DE-PS 27 03 076 und der GB-PS 15 76 608 beschriebene katalytische Hydrierung von Nitroarylsulfonsäuren mit Raney-Nickel in technischem Maßstab nicht wirtschaftlich durchführbar ist, weil der Katalysatorverbrauch zu hoch ist und hohe Ausbeuten an Aminoarylsulfonsäuren nur auf Kosten der Raum/Zeit-Ausbeute erreichbar sind bzw. bei den für ein wirtschaftliches Verfahren erforderlichen Raum/Zeit-Ausbeuten die Aminoarylsulfonsäuren nur in unzureichenden Ausbeuten erhalten werden. Außerdem wurde gefunden, daß die vorbeschriebenen Verfahren unter technischen Bedingungen keine reproduzierbaren Ergebnisse liefern.

Es wurde jetzt gefunden, daß die katalytische Hydrierung von Nitroarylsulfonsäuren in wäßrigen Medien mit Raney-Nickel oder Raney-Nickel/Eisen in hohen Ausbeuten bei gleichzeitig niedrigem, wirtschaftlich annehmbarem Katalysatorverbrauch und in den erforderlichen hohen Raum/Zeit-Ausbeuten gelingt, wenn man in den bekannten Verfahren die Feinverteilung des für die Reduktion benötigten Wasserstoffs in den wäßrigen Katalysator-Suspensionen wesentlich erhöht und gleichzeitig die Konzentrationen der zu hydrierenden Nitroarylsulfonsäure-Lösungen bzw. -Suspensionen, die zu den wäßrigen Katalysator-Suspensionen zudosiert werden, niedriger wählt, als dies bislang üblich war. Es wurde gefunden, daß durch die Kombination beider Maßnahmen - Erhöhung der Feinverteilung des Wasserstoffs und Herabsetzung der Konzentrationen der zu hydrierenden Nitroarylsulfonsäuren in den wäßrigen Katalysator-Suspensionen - eine wesentlich glattere Hydrierung der Nitroarylsulfonsäuren zu den Aminoarylsulfonsäuren (= höhere (Raum/Zeit)-Aus- beuten an Aminoarylsulfonsäuren) bei gleichzeitig verbessertem Erhalt der Aktivität des Katalysators (geringerer Katalysatorverbrauch) erreicht wird.

Die Erfindung betrifft daher ein Verfahren zur Herstellung von Aminoarylsulfonsäuren durch katalytische Hydrierung von Nitroarylsulfonsäuren, die in die Hydrierung in Form von Lösungen oder Suspensionen mit einer Konzentration von 0,45-0,55 Mol je kg eingeführt werden, mit Wasserstoff in Gegenwart von Raney-Nickel oder Raney-Nickel/Eisen in wäßrigen Medien unter einem Wasserstoffdruck von 100 bis 300 bar und bei Temperaturen von 130 bis 160°C, das dadurch gekennzeichnet ist, daß man den Wasserstoff in den wäßrigen Katalysator-Suspensionen so fein verteilt, daß die primäre Oberfläche der Gasblasen mehr als 60.000 $m^2/m^3$ $H_2$ beträgt.

Die erfindungsgemäß vorzunehmende Feinverteilung des Wasserstoffs in den wäßrigen Katalysator-Suspensionen auf eine Oberfläche >60.000 $m^2/m^3$ $H_2$, vorzugsweise auf Oberflächen (= Gas/Flüssig-Grenzflächen) von 600.000 bis 6 Mio $m^2/m^3$ $H_2$, entsprechend einem Primär-Gasblasendurchmesser von maximal 100 µm, vorzugsweise von 10 bis 1 µm, kann auf verschiedene, an sich bekannte Weisen vorgenommen werden; z.B. durch Einleiten von Wasserstoff unter Druck in die von einem hochtourigen Spezial-Rührer, z.B. einem Rohrrührer (Tourenzahl im 71-Laborautoklav >2.000 UpM), bewegten Katalysator-Suspensionen oder durch Einleiten des Wasserstoffs in das Druckgefäß mittels Strahldüse (Zweistoffdüse).

Die Feinverteilung des Wasserstoffs in den wäßrigen Katalysator-Suspensionen durch Einleiten mittels Strahldüse hat sich als besonders vorteilhaft erwiesen, weil sie erlaubt, auf die Verwendung bewegter Teile (Rührer) im Druckgefäß zu verzichten und weil man außerdem die wäßrigen Lösungen bzw. Suspensionen der zu hydrierenden Nitroarylsulfonsäuren oder, bei der kontinuierlichen Hydrierung, mit den wäßrigen Lösungen bzw. Suspensionen der Nitroarylsulfonsäuren vermischte wäßrige Katalysator-Suspensionen als Treibflüssigkeiten für die Strahldüsen verwenden und auf diese Weise das Einleiten und Feinverteilen des Wasserstoffs in den wäßrigen Katalysator-Suspensionen mit dem Zudosieren der zu hydrierenden Nitroverbindungen bzw. der kontinuierlichen Zufuhr von Katalysator-Suspensionen und Nitroarylsulfonsäuren kombinieren kann.

Als Vertreter der erfindungsgemäß zu hydrierenden Nitroarylsulfonsäuren seien beispielsweise genannt: 1-Nitronaphthalin-3,6,8-trisulfonsäure (Nitro-T-Säure; in reiner Form oder in Form des bei der Naphthalintrisulfierung und anschließenden Nitrierung anfallenden Nitronaphthalin-trisulfonsäure-Gemisches), 3-Nitronaphthalin-1,5-disulfonsäure (Nitro-Armstrong-Säure), 1-Nitronaphthalin-8-sulfonsäure (Nitro-Peri-Säure),

1-Nitronaphthalin-5-sulfonsäure (Nitro-Laurent-Säure),
3-Nitrobenzolsulfonsäure.

Aus den angegebenen Nitroarylsulfonsäuren werden im erfindungsgemäßen Verfahren folgende Aminosulfonsäuren erhalten:
1-Aminonaphthalin-3,6,8-trisulfonsäure (T-Säure),
3-Aminonaphthalin-1,5-disulfonsäure (C-Säure),
1-Aminonaphthalin-8-sulfonsäure (Peri-Säure),
1-Aminonaphthalin-5-sulfonsäure (Laurent-Säure) und
3-Aminobenzolsulfonsäure.

Ein wesentlicher Parameter des erfindungsgemäßen Verfahrens sind die Konzentrationen der Lösungen der Nitroarylsulfonsäuren, die für die Hydrierung eingesetzt werden. Es wurde gefunden, daß die Lösungen oder Suspensionen dieser Nitroarylsulfonsäuren bestimmte, für die einzelnen Nitroarylsulfonsäuren unterschiedliche Grenzkonzentrationen nicht überschreiten dürfen. In der nachfolgenden Tabelle sind diese Grenzkonzentrationen und die im erfindungsgemäßen Verfahren bevorzugt angewendeten Konzentrationsbereiche für die einzelnen Nitroarylsulfonsäuren zusammengestellt. Diese Grenzkonzentrationen werden durch Vorver-suche bestimmt.

Tabelle

| Nitroarylsulfonsäure | Grenzkonzentration [Mol/kg*] | [Gew.-%] | bevorzugter Konzentrationsbereich [Mol/kg] | [Gew.-%] |
|---|---|---|---|---|
| Nitro-T-Säure | 0,48 | 19,8 | 0,4-0,46 | 16,5-19 |
| Nitronaphthalin-trisulfon-säure-Gemisch MG 413 | | | | |
| Nitro-Armstrong-Säure | 0,55 | 18,3 | 0,45-0,5 | 15-17 |
| Nitro Peri/Laurent-Säure | 0,52 | 13,2 | 0,45-0,5 | 11-13 |
| 3-Nitrobenzolsulfonsäure | 0,45 | 9,2 | 0,40-0,44 | 8-9 |

* Lösung bzw. Suspension

Das erfindungsgemäße Verfahren kann sowohl diskontinuierlich als auch kontinuierlich durchgeführt werden.

Bei diskontinuierlicher Arbeitsweise kann z.B. wie folgt vorgegangen werden:

In einem mit einem hochtourigen Rührer (z.B. einem Rohr-Rührer) und Heiz- und Kühlelementen ausgestatteten Druckgefäß (Autoklaven) werden Raney-Nickel-Katalysator und Wasser vorgelegt. Nach Verdrängen der Luft aus dem Autoklaven durch aufeinanderfolgendes Spülen der Apparatur mit Stickstoff und Wasserstoff wird Wasserstoff bis zu einem Druck von 50 bar in den Autoklaven gedrückt. Der Inhalt des Druckgefäßes wird unter Rühren mit normaler Drehzahl (etwa 500 UpM) schnell auf 150°C erhitzt. Dann wird der Wasserstoffdruck im Autoklaven auf 150 bar eingestellt, die Rührgeschwindigkeit auf 2.500 UpM erhöht und die Lösung (Suspension) der zu hydrierenden Nitroarylsulfonsäure mit einer Geschwindigkeit, die in etwa der Hydriergeschwindigkeit (= Geschwindigkeit mit der die Nitroarylsulfonsäure zur Aminoarylsulfonsäure hydriert wird) entspricht, zudosiert. Der Druck im Autoklaven steigt während des Zudosierens der Nitroarylsulfonsäure auf etwa 200 bar an, die Temperatur des Autoklaveninhalts wird durch zeitweiliges Kühlen auf 150°C gehalten. Nach beendeter Zugabe der Nitroarylsulfonsäure wird das Hydriergemisch noch eine kurze Zeit, etwa 5 bis 10 Min., unter dem angegebenen Druck und der angegebenen Temperatur mit hoher Drehzahl gerührt. Dann wird die Drehzahl wieder auf eine normale Drehzahl (etwa 500 UpM) gesenkt und der Autoklaveninhalt auf 50°C abgekühlt. Der Rührer wird dann zum Absetzen des Katalysators abgestellt. Nach dem Entspannen des Druckgefäßes wird die katalysatorfreie wäßrige Lösung der Aminoarylsulfonsäure bis auf eine zum Suspendieren des abgesetzten Katalysators für den nächsten Ansatz erforderliche Menge aus dem Druckgefäß abgezogen und in üblicher Weise aufgearbeitet. Die im Druckgefäß zurückbleibende Suspension des gebrauchten Katalysators in der wäßrigen Lösung der Aminoarylsulfonsäure wird, gegebenenfalls nach Zusatz von 1 bis 2 Gew.-% frischem Katalysator, bezogen auf das Gewicht des gebrauchten Katalysators, als Vorlage für den nächsten Hydrier-Ansatz verwendet.

Es können zwei oder mehrere der vorstehend beschriebenen, diskontinuierlich arbeitenden Reaktoren zu einer Reaktorkaskade hintereinander geschaltet werden.

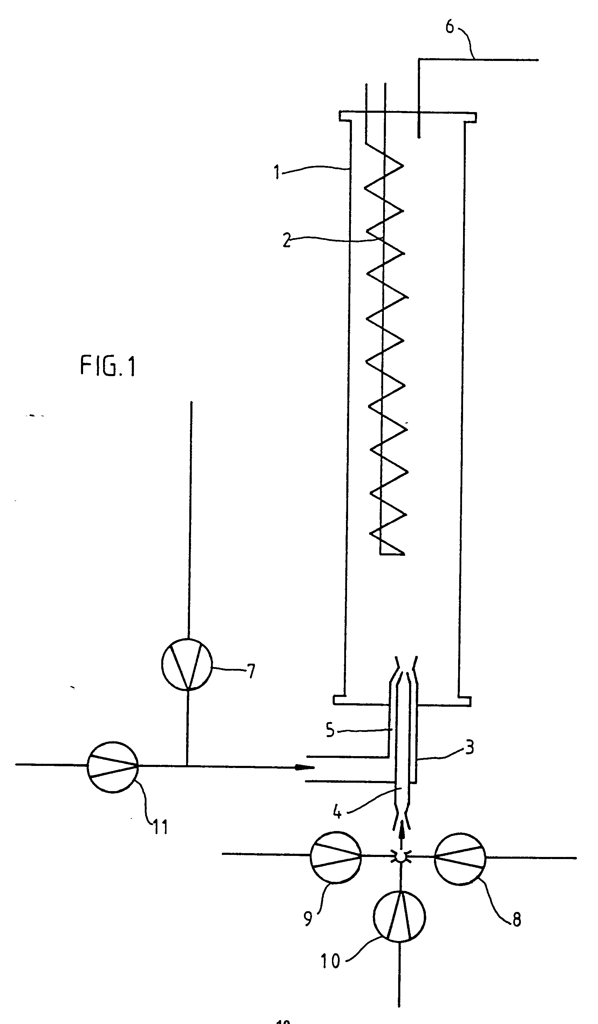

Die kontinuierliche Durchführung des erfindungsgemäßen Verfahrens kann z.B. in dem in Fig. 1 schematisch dargestellten, mit einer Heiz-/Kühl-Vorrichtung (2) und einer Strahldüse (3) versehenem Druckreaktor (1) vorgenommen werden. Durch das Innenrohr (4) der Strahldüse werden die flüssigen Reaktionskomponenten, die Katalysator-Suspension (im Kreislauf geführte Katalysator-Suspension und in Wasser suspendierter frischer Katalysator) und die Lösung bzw. Suspension der zu hydrierenden Nitroarylsulfonsäuren, durch das das Innenrohr (4) konzentrisch umgebende Außenrohr (5) der Strahldüse der Wasserstoff (Frisch-Wasserstoff und im Kreislauf geführter Wasserstoff) in den Reaktor (1) eingeleitet. Die mit feinverteiltem Wasserstoff gesättigte Suspension des Katalysators in der wäßrigen Lösung der Aminoarylsulfonsäuren wird über die Leitung (6) kontinuierlich aus dem Reaktor (1) abgezogen.

Nach dem Auftrennen dieser kontinuierlich aus dem Reaktor (1) abgezogenen Suspension in Wasserstoff, wäßrige Lösung der Aminoarylsulfonsäure und aufkonzentrierte Suspension des Katalysators in wäßriger Aminoarylsulfonsäure-Lösung, wird der zurückgewonnene Wasserstoff (Kreislauf-Wasserstoff) mittels Umwälzkompressor (7), die aufkonzentrierte Suspension des gebrauchten Katalysators in der wäßrigen Aminoarylsulfonsäure-Lösung nach Ausschleusen einer gewissen Menge an verbrauchtem Katalysator mittels Hochdruckpumpe (8) zusammen mit der über Hochdruckpumpe (9) zudosierten Frisch-Katalysator-Suspension und der über Hochdruckpumpe (10) zudosierten wäßrigen Suspension oder Lösung der zu hydrierenden Nitroarylsulfonsäure durch die Strahldüse (3) in den Hydrier-Reaktor (1) zurückgeführt bzw. eindosiert (Frischprodukte).

Bei der beschriebenen Arbeitsweise findet die Feinstverteilung des Wasserstoffs in der wäßrigen Katalysator-Suspension erst nach deren Vermischen mit der wäßrigen Lösung bzw. Suspension der Nitroarylsulfonsäure statt. Weil der Zeitraum bis zur Feinstverteilung des Wasserstoffs in der mit der Nitroarylsulfonsäure vermischten Katalysator-Suspension jedoch nur äußerst kurz ist und das Vermischen von wäßriger Katalysator-Suspension mit der wäßrigen Lösung bzw. Suspension der Nitroarylsulfonsäure bei Temperaturen von 30 bis 60°C, d.h. weit unterhalb der Hydriertemperatur, vorgenommen wird, tritt auch bei dieser Arbeitsweise kein Aktivitätsverlust des Katalysators ein.

Der Hydrier-Reaktor wird unter einem Wasserstoffdruck von 100 bis 200 bar betrieben. Die Kompressoren bzw. Hochdruckpumpen (7) bis (11) und die Abmessungen der Strahldüse sind so eingestellt, daß die erfindungsgemäße Feinverteilung des Wasserstoffs in der Suspension des Katalysators in der wäßrigen Lösung des Hydriergemisches auf >60.000 $m^2$ primäre Gasoberfläche/$m^3$ $H_2$ eingehalten wird. Bevorzugt wird mit einer Feinverteilung des Wasserstoffs in den Katalysator-Suspensionen von 600.000 bis 6 Mio $m^2$ primäre Gasoberfläche/$m^3$ $H_2$ gearbeitet.

Es können auch mehrere, z.B. drei der vorstehend beschriebenen Hydrier-Reaktoren (1) zu einer Reaktorkaskade hintereinander geschaltet werden.

Als Raney-Nickel-Katalysatoren werden im erfindungsgemäßen Verfahren die bekannten Raney-Nickel- und Raney-Nickel/Eisen-Katalysatoren verwendet.

Die Katalysatoren werden bei kontinuierlichen Hydrierungen von Nitroarylsulfonsäuren zu Aminoarylsulfonsäuren in üblichen Mengen von 1 bis 5 Gew.-%, bezogen auf das Gewicht der zu hydrierenden Nitroarylsulfonsäure-Losungen, eingesetzt. 1 bis 4, vorzugsweise 1,5 bis 2,5 Gew.-% dieser Katalysatormenge sollte aus frischem Katalysator bestehen, der Rest, 98,5 bis 97,5 Gew.-% aus gebrauchtem Katalysator.

Bei diskontinuierlichen Hydrierungen werden vorzugsweise Katalysator-Konzentrationen von ca. 0,2-1 Gew.-%, bezogen auf das Gewicht der zu hydrierenden Nitroarylsulfonsäure-Lösungen, eingesetzt, wobei zahlreiche Chargen (siehe Beispiele) an einem Katalysator-Einsatz hydriert werden können. (Die Anzahl der Chargen einer Kampagne kann auch hier durch Nachdosieren kleiner Frischkatalysator-Mengen erhöht werden).

Die wäßrigen Lösungen bzw. Suspensionen der zu hydrierenden Nitroarylsulfonsäuren sollten einen pH-Wert im Bereich von 7 bis 9,5 aufweisen.

Die erfindungsgemäße Hydrierung wird bei Temperaturen von 100 bis 180°C, vorzugsweise 130 bis 160°C, und Drucken von 100 bis 300 bar, vorzugsweise 130 bis 200 bar, vorgenommen.

Beispiel 1

Die Hydrierung wurde in dem in Fig. 1 schematisch dargestellten Hydrier-Reaktor vorgenommen (Innendurchmesser des Reaktors: 400 mm; Höhe: 6.900 mm; das Reaktor-Arbeitsvolumen betrug infolge der Einbauten zum Heizen bzw. Kühlen nur etwa 730 l).

Mit der Strahldüse wurde eine primäre Feinverteilung des Wasserstoffs in der wäßrigen Katalysator-Suspension von 600.000 bis 6 Mio m$^2$/m$^3$ H$_2$ eingestellt. Es wurden stündlich durch die Hochdruckpumpe (10)3,5 m$^3$ (etwa 4,03 t) einer Lösung in den Reaktor (1) gepumpt, die 0,42 Mol = 17,4 Gew.-% eines 1-Nitronaphthalin-trisulfonsäure-Gemisches folgender Zusammensetzung

82 Gew.-% 1-Nitronaphthalin-3,6-8-trisulfonsäure,

11 Gew.-% 1-Nitronaphthalin-3,5,7-trisulfonsäure,

4,5 Gew.-% 1-Nitronaphthalin-4,6,8-trisulfonsäure,

1,7 Gew.-% 1-Nitronaphthalin-2,5,7-trisulfonsäure und

etwa 1 Gew.-% 2-Nitronaphthalin-3,5,7-trisulfonsäure

je kg Lösung enthielt. (pH-Wert der Lösung: 7,5 bis 8). Gleichzeitig mit der Nitronaphthalintrisulfonsäure-Lösung wurden stündlich durch die Hochdruckpumpe (8) 1,7 m$^3$ der durch Leitung (6) abgezogenen und auf einen Gehalt von etwa 6 Gew.-% Raney-Nickel aufkonzentrierten Suspension des gebrauchten Katalysators in der wäßrigen Aminonaphthalinsulfonsäure-Losung und etwa 2,8 kg frisches, in Wasser suspendiertes Raney-Nickel durch die Hochdruckpumpe (9) eingepumpt.

Die Temperatur im Reaktor betrug 150°C, der Druck 200 bar. Es wurden je Stunde etwa 3.000 Nm$^3$ Wasserstoff umgewälzt und etwa 120 Nm$^3$ Frisch-Wasserstoff eingespeist. Der Umsatz an Nitroverbindungen betrug über 99,5 %, die Ausbeute an Aminonaphthalintrisulfonsäuren 98 % der Theorie. Der Katalysator-Verbrauch betrug 0,4 Gew.-%, bezogen auf das Gewicht der Nitronaphthalintrisulfonsäuren.

Wurde die Konzentration der Nitronaphthalintrisulfonsäuren von 0,42 Mol/kg Lösung = 17,4 Gew.-% auf 19,2 Gew.-% erhöht, so stieg der Katalysator-Verbrauch bereits auf das 1,5-fache, d.h., auf 0,6 Gew.-% an. Bei einer weiteren Steigerung der Konzentration der Nitronaphthalinsulfonsäure-Lösung erhöhte sich der Katalysator-Verbrauch überproportional und die Aufkonzentration und Abtrennung des Katalysators aus der Hydrier-Lösung wurde wegen Verklebens des Katalysators zunehmend schwieriger.

Beispiel 2

Als Hydrierapparatur wurde ein 7-1-V4A-Autoklav verwendet, der mit einem Rohrrührer für Begasungen (Drehzahl bis 2.500 UpM), einer Innenschlange für Dampfheizung bzw. Wasserkühlung, einem verkürzten Steigrohr (für Teilentleerung) und einem langem (bis zum Autoklavenboden reichenden) Steigrohr für eine Totalentleerung ausgerüstet war.

In diesen Reaktor wurden 1.000 ml Wasser und 20 g Raney-Nickel (100 %ig) vorgelegt. Nach dem Spülen des Autoklaven mit Stickstoff und Wasserstoff, wurde der Wasserstoffdruck auf 50 bar erhöht und der Autoklaveninhalt bei einer Rührerdrehzahl von 500 UpM rasch auf 150°C erhitzt. Dann wurde der Druck mit Wasserstoff auf 150 bar eingestellt, die Rührerdrehzahl auf 2.500 UpM erhöht (erzielte Feinverteilung des Wasserstoffs in der Katalysatorsuspension ca. 100.000 bis 4 Mio. m$^2$ primäre Gasoberfläche/m$^3$ H$_2$) und eine 0,48 Mol/kg Suspension (= 16 Gew.-%) enthaltende Suspension von Nitro-Armstrong-Säure (Magnesiumsalz) (pH-Wert der Suspension: 9,5) innerhalb von 15 Min. in den Autoklaven gepumpt. Der Druck stellte sich auf etwa 200 bar ein; die Temperatur wurde durch gelegentliches Kühlen auf 150°C gehalten.

Nach erfolgter Zugabe der Nitro-Armstrong-Säure - Magnesiumsalz-Suspension - wurde noch 10 Min. bei der angegebenen Temperatur und dem angegebenen Druck mit der hohen Drehzahl gerührt und dann die Drehzahl auf 500 UpM zurückgenommen.

Der Autoklaveninhalt wurde auf 50°C abgekühlt und dann zum Absetzen des Katalysators 1 Stunde ohne Rühren stehengelassen. Danach wurde der Druck im Autoklaven auf 10 bar entspannt und die katalysatorfreie wäßrige Aminonaphthalindisulfonsäure-Lösung über das kurze Steigrohr aus dem Autoklaven gedrückt und in üblicher Weise aufgearbeitet.

Die im Autoklaven zurückbleibende Menge von 800 ml wäßriger Aminonaphthalindisulfonsäure-Lösung und Katalysator wurde als Vorlage für den nächsten Ansatz verwendet. Insgesamt wurden auf diese Weise mit den 20 g Raney-Nickel 16 Ansätze von Nitronaphthalindisulfonsäure in der angegebenen Weise hydriert. Die Ausbeute an C-Säure betrug 98,3 % der Theorie, bezogen auf eingesetzte Nitro-C-Säure.

Der Katalysator-Verbrauch betrug 0,26 Gew.-%, bezogen auf eingesetzte Nitro-Verbindung.

Der Gehalt der einzelnen Ansätze an nicht reduzierter Nitro-Verbindung lag beim ersten Ansatz bei etwa 0,5 g/l und stieg bis zum sechzehnten Ansatz auf etwa 2,5 g/l an.

Der pH-Wert der wäßrigen Lösungen lag bei den Ansätzen nach der Hydrierung bei 8,5.

Die Verwendung einer höher konzentrierten Suspension von Nitro-Armstrong-Säure-Magnesiumsalz ergab eine wesentlich geringere Ausbeute (nur etwa 82 % der Theorie) und die Aktivität des Katalysators ließ sehr schnell nach.

Beispiel 3

Es wurde die in Beispiel 2 beschriebene Druckapparatur verwendet und nach der in Beispiel 2 beschriebenen Arbeitsweise verfahren. Nur wurden im Autoklaven 1.000 ml Wasser und 14 g Raney-Nickel/Eisen (85/15) (100 %ig) vorgelegt und eine wäßrige Lösung eines Gemisches aus Nitro-Peri-Säure und Nitro-Laurent-Säure, die je kg 0,46 Mol Nitro-Peri/Laurent-Säure (= 12 Gew.-%) enthielt, in den Autoklaven eingepumpt.

Mit den 14 g Raney-Nickel/Eisen konnten 12 Ansätze = 5.174 g (= 20,45 Mol) Nitro-Peri/Laurent-Säure hydriert werden. Die Ausbeute an Peri/Laurent-Säure betrug 4.514 g (= 20,24 Mol) entsprechend einer Ausbeute von 99 % der Theorie, bezogen auf eingesetzte Nitro-Peri/Laurent-Säure.

Der Katalysator-Verbrauch betrug 0,27 Gew.-%, bezogen auf eingesetzte Nitro-Verbindung.

Beispiel 4

Es wurde die in Beispiel 2 beschriebene Druckapparatur verwendet und nach der in Beispiel 2 beschriebenen Arbeitsweise verfahren.

Für die Hydrierung wurden 15 g Raney-Nickel/Eisen (85/15) eingesetzt und eine 0,4 Mol/kg Lösung (8 gew.-%ige) wäßrige Lösung von 3-Nitrobenzolsulfonsäure (in Form ihres Na-Salzes) in den Autoklaven unter den in Beispiel 2 beschriebenen Bedingungen eingepumpt.

Mit den 15 g Raney-Nickel/Eisen konnten 23 Ansätze = 7.475 g (= 36,8 Mol) 3-Nitrobenzolsulfonsäure hydriert werden.

Die Ausbeute an Metanilsäure betrug 6.315 g = 36,5 Mol entsprechend 99,2 % der Theorie, bezogen auf eingesetzte Nitrobenzolsulfonsäure.

Der Katalysator-Verbrauch betrug 0,2 Gew.-%, bezogen auf eingesetzte 3-Nitrobenzolsulfonsäure.

Der Gehalt der in den einzelnen Ansätzen erhaltenen Metanilsäure an nicht umgesetzter Nitrobenzolsulfonsäure lag im Durchschnitt bei 0,02 Gew.-% und stieg beim 22. und 23. Ansatz auf 0,6 bzw. 0,8 Gew.-%.

Wegen geringer Mengen an sulfidischen Verunreinigungen in der 3-Nitrobenzolsulfonsäure wurde diese vor ihrer Hydrierung mit Wasserstoffperoxid in folgender Weise vorbehandelt:

Die Lösung von 2.000 g 3-Nitrobenzolsulfonsäure-Natriumsalz in 8.000 g entsalztem Wasser wurde bei 90°C mit etwa 20 g 50 %iger Natronlauge auf einen pH-Wert von 10 eingestellt, dann tropfenweise innerhalb von etwa 30 Min. mit 65 g Wasserstoffperoxid-Lösung (17,5 %ig) versetzt, 15 Min. gerührt und abschließend mit 15 g 50 %iger Schwefelsäure auf einen pH-Wert von 8,0 eingestellt.

**Patentansprüche**

1.  Verfahren zur Herstellung von Aminoarylsulfonsäuren durch katalytische Hydrierung von Nitroarylsulfonsäuren, die in die Hydrierung in Form von Lösungen oder Suspensionen mit einer Konzentration von 0,45 - 0,55 Mol je kg eingeführt werden, mit Wasserstoff in Gegenwart von Raney-Nickel oder Raney-Nickel/Eisen in wäßrigen Medien unter einem Wasserstoffdruck von 100 bis 300 bar und bei Temperaturen von 130 bis 160°C, dadurch gekennzeichnet, daß man den Wasserstoff in den wäßrigen Katalysator-Suspensionen so fein verteilt, daß die primäre Oberfläche der Gasblasen mehr als 60.000 $m^2/m^3$ $H_2$ beträgt.

2.  Verfahren nach Anspruch 1, dadurch gekennzeichnet, daß der Wasserstoff in den wäßrigen Katalysator-Suspensionen so verteilt wird, daß die primär erzielte Oberfläche (Gas-Flüssig-Grenzfläche) 600.000 bis 6 Mio $m^2/m^3$ $H_2$ beträgt.

3.  Verfahren nach Anspruch 1 oder 2, dadurch gekennzeichnet, daß der Gehalt der Lösungen oder Suspensionen für die einzelnen Nitroarylsulfonsäuren
    < 0,48 Mol Nitro-T-Säure,
    < 0,55 Mol Nitro-Armstrong-Säure,

< 0,52 Mol Nitro-Peri/Laurent-Säure
oder < 0,45 Mol 3-Nitrobenzol-sulfonsäure
je kg Lösung (Suspension) beträgt.

4. Verfahren nach Anspruch 1 oder 2, dadurch gekennzeichnet, daß der Gehalt der Lösungen oder Suspensionen für die einzelnen Nitroarylsulfonsäuren
0,4 - 0,46 Mol Nitro-T-Säure,
0,45 - 0,5 Mol Nitro-Armstrong-Säure
0,45 - 0,5 Mol Nitro-Peri/Laurent-Säure oder
0,40 - 0,44 Mol 3-Nitrobenzolsulfonsäure
je kg Lösung (Suspension) beträgt.

## Claims

1. Process for preparing aminoarylsulphonic acids by catalytic hydrogenation of nitroarylsulphonic acids, which are introduced into the hydrogenation in the form of solutions or suspensions having a concentration of 0.45 - 0.55 mol per kg, with hydrogen in the presence of Raney nickel or Raney nickel/iron in aqueous media under a hydrogen pressure of from 100 to 300 bar and at temperatures of from 130 to 160°C, characterized in that the hydrogen is so finely divided in the aqueous catalyst suspensions that the primary surface area of the gas bubbles is greater than 60,000 $m^2/m^3$ of $H_2$.

2. Process according to Claim 1, characterized in that the hydrogen in the aqueous catalyst suspensions is divided so that the primary surface area achieved (gas-liquid interface) is from 600,000 to 6 million $m^2/m^3$ $H_2$.

3. Process according to Claim 1 or 2, characterized in that the individual nitroarylsulphonic acid contents of the solutions or suspensions are
< 0.48 mol of nitro-T acid,
< 0.55 mol of nitro-Armstrong acid,
< 0.52 mol of nitro-Peri/Laurent acid
or <0.45 mol of 3-nitrobenzenesulphonic acid
per kg of solution (suspension).

4. Process according to Claim 1 or 2, characterized in that the individual nitroarylsulphonic acid contents of the solutions or suspensions are
0.4 - 0.46 mol of nitro-T acid,
0.45 - 0.5 mol of nitro-Armstrong acid,
0.45 - 0.5 mol of nitro-Peri/Laurent acid or
0.40 - 0.44 mol of 3-nitrobenzenesulphonic acid
per kg of solution (suspension).

## Revendications

1. Procédé pour préparer des acides aminoarylsulfoniques par hydrogénation catalytique d'acides nitroarylsulfoniques envoyés à l'hydrogénation à l'état de solutions ou suspensions à une concentration de 0,45 à 0,55 mol/kg, en présence de nickel de Raney ou de nickel/fer de Raney, en milieu aqueux, sous une pression d'hydrogène de 100 à 300 bar et à des températures de 130 à 160°C, caractérisé en ce que l'on répartit l'hydrogène dans la suspension aqueuse de catalyseur avec une finesse telle que la surface primaire des bulles de gaz est supérieure à 60 000 $m^2/m^3$ de $H_2$.

2. Procédé selon la revendication 1, caractérisé en ce que l'on répartit l'hydrogène dans la suspension aqueuse de catalyseur à une finesse telle que la surface primaire obtenue (surface limite gaz-liquide) est de 600 000 à 6 millions de $m^2/m^3$ de $H_2$.

3. Procédé selon la revendication 1 ou 2, caractérisé en ce que la teneur des solutions ou suspensions en les acides nitroarylsulfoniques indivuels est de :
moins de 0,48 mol d'acide nitro-T

moins de 0,55 mol d'acide nitro-Armstrong
moins de 0,52 mol d'acide nitro-Peri/Laurent, ou
moins de 0,45 mol d'acide 3-nitrobenzène-sulfonique,
par kg de solution (suspension).

4. Procédé selon la revendication 1 ou 2, caractérisé en ce que la teneur des solutions ou suspensions en les acides nitroarylsulfoniques individuels est de :
0,4 à 0,46 mol d'acide nitro-T
0,45 à 0,5 mol d'acide nitro-Armstrong
0,45 à 0,5 mol d'acide nitro-Peri/Laurent ou
0,40 à 0,44 mol d'acide 3-nitrobenzène-sulfonique,
par kg de solution (suspension).

FIG.1